# FASCICULE DE BREVET EUROPEEN

(11) **EP 2 209 006 B1**
(45) Date de publication et mention de la délivrance du brevet: **16.12.2015**
(21) Numéro de dépôt: 10150607.9
(22) Date de dépôt: 13.01.2010
(51) Int. Cl.: G01N 33/68, A61Q 19/08, A61K 8/64, A61K 38/17

(54) **Utilisation à des fins de criblage d'actifs anti-âges de formes solubles de la protéine de type Desmogléine I**
Anwendung der löslichen Formen des Proteins vom Typ Desmoglein I zum Screening von Anti-Age-Aktivstoffen
Use of the soluble forms of the Desmoglein I protein for screening anti-ageing active ingredients

(30) Priorité: 13.01.2009 FR 0950159; 27.01.2009 US 202078 P
(43) Date de publication de la demande: 21.07.2010
(73) Titulaire: L'Oréal, 75008 Paris (FR)
(72) Inventeur: Donovan, Mark, 95810 Berville (FR); Simonetti, Lucie, 94300 Vicennes (FR); Bernard, Dominique, 92170 Vanves (FR)
(74) Mandataire: Le Coupanec, Pascale A.M.P.

(56) Documents cités:
- WO-A2-02/053773
- WO-A2-2009/081368
- FR-A1- 2 925 312
- JP-A- 2006 006 143
- JP-A- 2006 008 570
- BARTOLONE J; DOUGHTY D; EGELRUD T: "A NON-INVASIVE APPROACH FOR ASSESSING CORNEOCYTE COHESION IMMUNOCHEMICAL DETECTION OF DESMOGLEIN I" CLINICAL RESEARCH, THOROFARE, NJ, US, vol. 39, no. 2, 1 janvier 1991 (1991-01-01), page 471A, XP009105453 ISSN: 0009-9279
- HARATAKE A; ET AL: "ACCELERATION OF DE NOVO CHOLESTEROL SYNTHESIS IN THE EPIDERMIS INFLUENCES DESQUAMATION OF THE STRATUM CORNEUM IN AGED MICE" SKIN PHARMACOLOGY AND PHYSIOLOGY: JOURNAL OF PHARMACOLOGICAL ANDBIOPHYSICAL RESEARCH, S. KARGER AG, BASEL, CH, vol. 19, no. 5, 1 août 2006 (2006-08-01), pages 275-282, XP002501138 ISSN: 1660-5527 [extrait le 2006-06-16]

## Description

Le présent brevet décrit l'utilisation de formes peptidiques solubles, complexées ou non, dérivant au moins en partie de la Desmogléine I, pour caractériser l'efficacité d'actifs potentiels à l'égard d'un épiderme manifestant un ou plusieurs signes de vieillissement cutané.

Les épithéliums sont des tissus dont les cellules sont jointives et solidaires les unes des autres et reposent sur une membrane basale. Ils forment un revêtement soit externe, par exemple en surface de la peau, ou l'épiderme, soit interne, en surface d'une muqueuse. Ils peuvent également former des glandes.

Plus précisément, ces épithéliums sont des structures dont l'homéostasie résulte de la mise en oeuvre d'un ensemble, finement régulé, de signaux intracellulaires et extracellulaires agissant à toutes les étapes de la prolifération, de la migration, de la différenciation cellulaire, ainsi que de la synthèse des différents composants de la matrice extracellulaire. Ces signaux peuvent, notamment, résulter de l'action de facteurs produits par des kératinocytes.

Le maintien des bonnes fonctions physiologiques d'un épithélium implique notamment la différenciation épithéliale terminale et/ou la synthèse de protéoglycannes.

En ce qui concerne plus particulièrement l'épiderme, il s'agit d'un épithélium, conventionnellement divisé en une couche basale de kératinocytes contenant, notamment, des cellules souches cutanées et constituant la couche germinative de l'épiderme, une couche dite épineuse constituée de plusieurs couches de cellules polyédriques disposées sur la couche basale, une couche dite granuleuse comprenant une à trois couches dites de cellules aplaties contenant des inclusions cytoplasmiques distinctes, les grains de kératohyaline, et enfin, un ensemble de couches supérieures, appelé couche cornée (ou *stratum corneum*) constituée de kératinocytes au stade terminal de leur différenciation appelés cornéocytes.

Le *stratum corneum,* la partie la plus externe de la peau qui assure la fonction barrière entre l'organisme et l'environnement, et la tige pilaire, partie émergente du follicule pileux qui constitue la chevelure, représentent tous deux l'aboutissement du processus de différenciation des kératinocytes. La différenciation épidermique suit un processus de maturation dans lequel des kératinocytes de la couche basale se différencient et migrent pour aboutir à la formation des cornéocytes, cellules mortes totalement kératinisées. Cette différenciation est la résultante de phénomènes parfaitement coordonnés qui vont conduire au maintien d'une épaisseur constante et assurer ainsi l'homéostasie de l'épiderme.

De nombreux troubles ou pathologies cutanées peuvent résulter d'un dysfonctionnement de l'homéostasie de l'épiderme.

Dans le cas de la peau âgée, ce dysfonctionnement se manifeste généralement par l'apparition de rides (micro-relief et rides profondes), une perte de l'élasticité, un toucher rugueux et une sécheresse. Sur le plan histologique, un aplatissement de la jonction dermo-épidermique et une diminution d'épaisseur du derme et de l'épiderme sont observés. Le contenu en collagène et en glycosaminoglycannes diminue. La fonction barrière de la peau est altérée. L'ensemble de ces phénomènes est accru par l'exposition solaire chronique.

De même, ce dysfonctionnement peut être exacerbé chez les femmes lors de la ménopause.

Il est connu aujourd'hui que ces dysfonctionnements peuvent être notamment associés à une modulation différente de l'expression et/ou de l'activité biologique de certaines protéines exprimées dans le s*tratum corneum.* Telle est en particulier le cas de la Desmogléine I.

La Desmogléine I (encore dénommée DGI) est une glycoprotéine transmembranaire comprenant dans sa forme préproprotéine 1049 acides aminés et présentant un poids moléculaire d'environ 114 kDa à 150 kDa, selon qu'elle est ou non glycosylée. Outre des sites de glycosylation sur les résidus Asparagine en position 36, 110 et 180, la séquence de cette protéine comprend également des sites de liaison au calcium.

Cette protéine est apparentée aux cadhérines et appartient à une famille de protéines qui comprend également les Desmogléines II et III. La DGI est présente uniquement dans les desmosomes dont elle est un des éléments majeurs de structure.

Le document US 2004/0142335 rend compte, par le biais d'une analyse transcriptomique, d'une augmentation de l'expression de l'ARNm codant pour la protéine Desmogléine I dans une peau d'un individu âgé comparativement à celle d'un individu jeune. Ce document ne mentionne aucune forme peptidique soluble de la Desmogléine I.

Le document Haratake et al. (2006 ; Skin Pharmacol. Physiol. 19 :275-282) montre une augmentation de l'expresssion de la desmogléine I, au niveau protéique, dans la peau âgée d'un modèle murin.

Plus récemment, les inventeurs ont pour leur part, constaté une augmentation significative de l'expression de la Desmogléine I, et donc une accumulation de cette dernière dans *le stratum corneum*, au cours du vieillissement chronologique de l'épiderme. Le niveau d'expression de la Desmogléine I au niveau du *stratum corneum* constitue donc un indice utile pour caractériser l'état physiologique de la peau, notamment en terme de vieillissement.

La présente invention résulte pour sa part de la caractérisation par les inventeurs, au niveau d'un *stratum corneum* humain ayant subi un traitement de type desquamation, de formes peptidiques solubles, distinctes de la Desmogléine I mais dérivant manifestement de celle-ci. On peut penser que les formes peptidiques caractérisées par les inventeurs sont issues au moins en partie de la protéolyse de la Desmogléine I.

En particulier, une forme peptidique soluble de l'invention est distincte de la séquence entière de la Desmogléine I, à savoir SEQ ID NO 2.

Le document JP 2006/006143 décrit l'utilisation de peptides issus de la protéolyse de la desmogléine I pour cribler des activités de type « toxines exfoliatives ».

Comme il ressort de ce qui suit, ces formes peptidiques solubles, complexées ou non, de cette protéine ont été caractérisées par les inventeurs grâce à la mise au point d'une technique de dosage ELISA spécifique, notamment décrite dans l'exemple 1, ci-après.

En conséquence, ces formes solubles se révèlent être un outil particulièrement intéressant pour cribler des actifs anti-âge, voire caractériser l'efficacité d'un traitement pour prévenir et/ou traiter le vieillissement cutané notamment via une action, vraisemblablement de type dégradation, à l'égard de l'accumulation de la Desmogléine I au niveau du *stratum corneum,* constatée lors du vieillissement cutané.

Dans les deux cas, l'efficacité de l'actif ou du traitement considéré est vérifiée via une augmentation de la libération d'au moins une, voire de plusieurs des formes solubles considérées selon l'invention.

Le présent brevet décrit l'utilisation d'au moins une forme peptidique soluble, complexée ou non, dérivant au moins en partie d'un polypeptide de séquence d'acides aminés codée par une séquence d'acides nucléiques représentée par tout ou partie de SEQ ID NO 1, ou un analogue de celle-ci, à titre d'outil de criblage d'actifs utiles pour prévenir et/ou traiter le vieillissement cutané.

Selon l'invention, une forme peptidique soluble convenant à l'invention n'est pas représentée par la séquence SEQ ID NO 2.

Une augmentation de la quantité en ces formes peptidiques solubles est un indice de l'efficacité d'un traitement anti-âge.

En particulier, une détection d'une présence ou d'une augmentation d'une teneur en ladite/lesdites forme(s) peptidique(s) est indicative d'un actif doté de propriétés utiles pour prévenir et/ou traiter le vieillissement cutané.

L'invention est relative à un procédé *in vitro* ou *ex vivo* de criblage d'actifs comprenant au moins les étapes consistant à :
a) mettre en contact, au moins un type cellulaire apte à libérer au moins une forme soluble complexée ou non, dérivant de la protéolyse du polypeptide de séquence d'acides aminés représentée en SEQ ID NO 2, avec au moins un actif anti-âge à tester, dans des conditions propices à une libération de ladite forme peptidique soluble,
b) déterminer une teneur en ladite forme peptidique soluble, et
c) comparer ladite teneur déterminée à l'étape b) à une teneur de ladite forme soluble déterminée en absence de composé chimique ou biologique à tester,
dans lequel une détection ou une caractérisation d'une présence ou d'une augmentation d'une teneur de ladite forme peptidique soluble est significative d'une efficacité dudit actif.

L'invention concerne aussi un procédé *in vitro* ou *ex vivo* pour caractériser l'efficacité d'un traitement cosmétique ou thérapeutique visant, chez un individu, à prévenir et/ou traiter les signes de vieillissement cutané liés au vieillissement chronologique, comprenant au moins la caractérisation qualitative ou quantitative d'au moins une forme peptidique soluble complexée ou non, dérivant de la protéolyse du polypeptide de séquence d'acides aminés représentée en SEQ ID NO 2, dans lequel une détection ou une caractérisation d'une présence ou d'une augmentation d'une teneur de ladite forme peptidique soluble est significative d'une efficacité dudit traitement.

Avantageusement, il peut être effectué, en outre, à l'issue de l'étape c), une étape de sélection du ou des actifs pour lequel ou lesquels on observe une augmentation de la teneur en forme(s) soluble(s).

Le présent brevet décrit aussi un procédé, notamment cosmétique, non invasif, pour caractériser l'efficacité d'un traitement cosmétique ou thérapeutique visant, chez un individu, à prévenir et/ou traiter les signes de vieillissement cutané liés au vieillissement chronologique, tels que les rides et ridules comprenant au moins la caractérisation qualitative ou quantitative d'au moins une forme peptidique soluble de la Desmogléine I selon l'invention.

En particulier, selon le procédé de l'invention, une détection ou une caractérisation d'une présence ou d'une augmentation d'une teneur de ladite forme peptidique est significative d'une efficacité dudit actif ou dudit traitement.

L'un des procédés précédemment décrits peut comprendre, en outre, à l'issue de l'étape de caractérisation d'au moins une forme peptidique soluble de la Desmogléine I, au moins une étape complémentaire consistant à administrer à l'individu ainsi évalué, une composition de soin, notamment cosmétique, établie ou sélectionnée au regard de l'information obtenue sur ladite forme soluble. En particulier, ladite étape complémentaire peut être consécutive à l'étape de caractérisation.

Une telle composition peut être sélectionnée parmi une gamme de compositions, chacune étant adaptée à un type d'information susceptible d'être obtenu à l'issue de l'étape de caractérisation.

Ainsi, il est décrit, d'une part, un procédé simple et rapide pour caractériser un état physiologique d'un épithélium et en particulier de l'épiderme de la peau, et d'autre part, pour ajuster en conséquence un traitement adéquat audit épithélium ou épiderme.

Par signes de vieillissement cutané, on entend toutes les modifications de l'aspect extérieur de la peau dues au vieillissement chronologique, comme par exemple les rides et ridules, la peau flétrie, le manque d'élasticité et/ou de tonus de la peau, l'amincissement du derme et/ou la dégradation des fibres de collagène ce qui entraîne l'apparence de peau molle et ridée.

Plus précisément, le procédé, non thérapeutique, tel que défini ci-dessus, vise à caractériser l'efficacité d'un traitement susceptible de prévenir et/ou traiter les signes du vieillissement cutané chez un individu, comprenant au moins les étapes consistant à :
i. disposer d'au moins un premier échantillon de surface cutané représentatif dudit individu,
ii. quantifier au niveau dudit échantillon, notamment via une technique de dosage ELISA, en particulier comme décrite en exemple 1 ci-après, au moins une forme peptidique soluble complexée ou non, dérivant au moins en partie de la protéolyse du polypeptide de séquence d'acides aminés représentée en SEQ ID NO 2.
iii. renouveler les étapes i. et ii. sur un deuxième échantillon de surface cutané représentatif dudit individu, et
iv. comparer les résultats obtenus à l'issu des étapes ii. et iii., notamment afin d'en déduire une information relative à au moins un effet du traitement,
dans lequel la détection à l'étape iv) d'une présence ou d'une augmentation d'une teneur de ladite forme peptidique soluble est indicative d'une efficacité dudit traitement, et lesdits premier et second échantillons de surface cutané correspondant à des stades de traitement différents.

La valeur ou donnée de référence à l'étape ii. peut-être une donnée obtenue à partir de l'épithélium, notamment de l'épiderme représentatif de l'individu faisant l'objet du traitement, antérieurement à l'administration dudit traitement.

Selon un mode de réalisation préféré, le premier échantillon est représentatif d'un état pré-traitement et le second est représentatif d'un état en cours de traitement ou post-traitement.

Une détection à l'étape iv. d'une présence ou d'une augmentation d'une teneur de ladite forme peptidique soluble est significative d'une efficacité dudit traitement.

Selon encore un autre mode de réalisation, un procédé de l'invention peut, en outre, comprendre une étape consistant à ajuster chez ledit individu ledit traitement par administration d'une composition de soin cosmétique établie ou sélectionnée au regard de l'information obtenue à l'issue de l'étape iv..

Selon encore un autre aspect, la présente invention décrit également l'utilisation d'au moins une forme peptidique soluble conforme à l'invention, à titre d'outil de criblage de composés biologiques ou chimiques susceptibles d'agir vis-à-vis de la Desmogléine I et notamment de la dégrader, et par conséquent capable d'induire et de favoriser la libération des formes solubles de la Desmogléine I.

La caractérisation de ces formes solubles peut également s'avérer utile pour établir un diagnostic de l'état d'un épiderme.

Ainsi, le présent brevet décrit l'utilisation d'une forme peptidique soluble selon l'invention, à titre d'outil de caractérisation *in vitro* ou *ex vivo* d'un état d'un épithélium, notamment d'un état d'un vieillissement chronologique d'un épithélium, la détection d'une présence ou d'une diminution d'une teneur en ladite/lesdites forme(s) peptidique(s) étant indicative d'un état de vieillissement chronologique d'un épithélium.

Le présent brevet décrit, selon un autre de ses aspects, un procédé, notamment cosmétique, non invasif, pour caractériser, notamment *in vitro* ou *ex vivo*, l'état chronologique d'un épiderme, comprenant au moins la caractérisation qualitative ou quantitative de formes peptidiques solubles de la Desmogléine 1 selon l'invention.

Ce procédé de caractérisation d'un état d'un épithélium comprend au moins les étapes consistant à :
a) déterminer dans un échantillon de surface dudit épithélium, notamment via une technique de dosage ELISA, une teneur en au moins une forme peptidique soluble conforme à l'invention, et
b) comparer ladite teneur déterminée à l'étape a) à une valeur de référence.

Selon une variante de réalisation, la donnée ou valeur obtenue peut être appréciée comparativement à une donnée ou valeur de référence, obtenue par exemple à partir d'au moins un épithélium, notamment un épiderme, distinct de celui faisant l'objet de la caractérisation, et dont l'état est connu.

Les procédés de l'invention peut être effectués *in vitro* ou, *ex vivo*.

Comme il ressort de la description qui suit, les procédés selon l'invention est particulièrement avantageux dans la mesure où sa mise en oeuvre ne requiert pas d'opération invasive.

En effet, la localisation par les inventeurs de ces nouveaux biomarqueurs dans le *stratum corneum* rend possible une caractérisation quantitative ou qualitative de l'expression de ceux-ci par simple prélèvement topique.

De manière avantageuse, il peut être réalisé sur un échantillon de *stratum corneum* du sujet considéré, simplement prélevé par stripping. La méthode de prélèvement peut par exemple être une technique de type stripping consistant à appliquer sur l'épithélium considéré, tel qu'un épiderme, une portion de ruban adhésif. En décollant ce ruban adhésif, une fraction de l'épithélium, par exemple une fraction épidermique, est prélevée. Après extraction protéique, celle-ci est ensuite analysée par un test ELISA tel que considéré dans la présente invention.

Le présent brevet décrit également l'utilisation d'une quantité efficace de forme(s) peptidique(s) soluble(s) conforme(s) à l'invention, pour la préparation et/ou l'amélioration d'un modèle cellulaire pluristratifié, en particulier un modèle de peau reconstruite.

Au sens de la présente description, on entend désigner par l'expression « quantité efficace » la quantité minimale nécessaire à l'obtention de l'effet attendu.

Selon encore un autre aspect, la présente invention décrit un procédé de préparation d'une peau reconstruite isolée comprenant au moins l'étape consistant à mettre en contact au moins une forme peptidique soluble selon l'invention, avec des cellules susceptibles de générer une peau reconstruite isolée, et notamment des kératinocytes.

### Définition de «formes solubles» de la Desmogléine I

Au sens de la présente invention, le terme « soluble » entend qualifier la faculté de la forme peptidique, complexée ou non, et considérée selon l'invention, à se solubiliser dans l'eau ou dans un milieu aqueux sans substances dénaturante des protéines, tels que les agents chaotropiques ou les détergents ioniques par exemple, par opposition à la Desmogléine I native, uniquement extractible en présence de tels agents.

Au sens de la présente invention le terme « complexée » se réfère à un conjugué soluble d'une des formes peptidiques considérées selon l'invention, avec soit une protéine distincte de la Desmogléine I ou un fragment de cette protéine, soit une autre forme soluble de la protéine Desmogléine I ou un de ses dérivés.

Au sens de la présente invention, le terme « dérivé de la protéine Desmogléine I » désigne un fragment de celle-ci ou un analogue, tel que défini ci-après.

Ces formes complexées solubles peuvent être le produit résultant de l'association de la protéine Desmogléine I ou l'un de ses fragments, avec une protéine annexe, encore appelée protéine cible.

A titre d'illustration de ces protéines susceptibles d'interagir avec la Desmogléine I et/ou l'un de ses fragments, peuvent notamment être cités la Desmocolline 2a (Dsc2), les Plakophilines 1, 2 et 3, la Plakoglobine, la Kallikreine 5, l'hormone de croissance 1 (GH1), la SSSCA1 (Centromeric Autoantigen 27 kD), la RuvB-like 1, la protéine C3orf10, et la VRK3 (Vaccinia related kinase 3).

Comme précisé précédemment, les formes solubles conformes à l'invention dérivent, au moins en partie, de la protéolyse d'un polypeptide de séquence d'acides aminés codée par une séquence d'acides nucléiques représentée par tout ou partie de SEQ ID NO 1, ou un analogue de celle-ci.

Au sens de la présente invention, on entend désigner par « fragment de séquence d'acides nucléiques », une séquence d'acides nucléiques codant en partie le polypeptide à partir duquel dérivent les formes solubles conformes à l'invention, ou des analogues de celles-ci, et en particulier une séquence d'acides nucléiques représentée par SEQ ID NO 1 ou un analogue de celle-ci.

Par « analogue d'une séquence d'acides nucléiques », on entend désigner toute séquence d'acides nucléiques, éventuellement résultant de la dégénérescence du code des acides nucléiques, et codant en partie pour des formes solubles de séquence identique ou analogue à celles codée en partie par ladite séquence d'acides nucléiques.

Les séquences d'acides nucléiques peuvent être issues de toutes origines possibles, à savoir soit animale, en particulier de mammifères et encore plus particulièrement humaine, soit végétale, soit de micro-organismes (virus, phages, bactéries entre autres) ou encore de champignons, sans préjuger du fait qu'elles soient présentes de manière naturelle ou non dans ledit organisme d'origine.

Les formes solubles conformes à l'invention dérivent de la protéolyse du polypeptide de séquence d'acides aminés représentée en SEQ ID NO 2.

Au sens de la présente invention, on entend désigner de manière générale, sauf indication contraire, par la Desmogléine I, la séquence (SEQ ID NO 2) de la protéine ayant ou non subi des modifications post-traductionnelles, de type N-acétylglycosylation sur les résidus Asparagine en position 36, 110 ou 180, susceptibles de modifier son poids moléculaire apparent ou son point isoélectrique.

Il est par ailleurs connu que la séquence primaire d'un polypeptide, c'est-à-dire l'enchaînement des acides aminés, détermine des sites spécifiquement reconnus par des enzymes de type protéase, telles que la trypsine qui, une fois la reconnaissance de ces sites effective, vont induire le clivage du polypeptide par protéolyse. Cette protéolyse résulte en la génération de divers peptides, ou fragments de protéolyse qui, lorsqu'ils sont sous une forme soluble, s'avèrent représentatifs des formes peptidiques solubles de la Desmogléine I considérées selon l'invention.

Il est décrit également un polypeptide à partir duquel dérivent les formes solubles ayant une séquence d'acides aminés choisie parmi SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24, SEQ ID NO 25, SEQ ID NO 26, SEQ ID NO 27, SEQ ID NO 28, SEQ ID NO 29, SEQ ID NO 30, SEQ ID NO 31, SEQ ID NO 32, SEQ ID NO 33, SEQ ID NO 34, SEQ ID NO 35, SEQ ID NO 36, SEQ ID NO 37, SEQ ID NO 38, SEQ ID NO 39, SEQ ID NO 40, SEQ ID NO 41, SEQ ID NO 42, SEQ ID NO 43, SEQ ID NO 44, SEQ ID NO 45, SEQ ID NO 46, SEQ ID NO 47, SEQ ID NO 48, SEQ ID NO 49, SEQ ID NO 50, SEQ ID NO 51, SEQ ID NO 52, SEQ ID NO 53, SEQ ID NO 54, SEQ ID NO 55, SEQ ID NO 56, SEQ ID NO 57, SEQ ID NO 58, SEQ ID NO 59, SEQ ID NO 60, SEQ ID NO 61, SEQ ID NO 62, SEQ ID NO 63, SEQ ID NO 64, SEQ ID NO 65, SEQ ID NO 66, SEQ ID NO 67, SEQ ID NO 68, SEQ ID NO 69, SEQ ID NO 70, SEQ ID NO 71, SEQ ID NO 72, SEQ ID NO 73, SEQ ID NO 74, SEQ ID NO 75, SEQ ID NO 76, SEQ ID NO 77, SEQ ID NO 78, SEQ ID NO 79, SEQ ID NO 80, SEQ ID NO 81, SEQ ID NO 82, SEQ ID NO 83, SEQ ID NO 84 et SEQ ID NO 85, et leurs mélanges.

Par « analogue d'un polypeptide », on entend désigner tout polypeptide présentant une homologie de séquence, en particulier vis-à-vis d'une des séquences caractéristiques dudit polypeptide, ainsi qu'une activité biologique de même nature. Cet analogue peut être un agent peptidomimétique.

L'homologie peut être d'au moins 85 %, par exemple d'au moins 90 %, et par exemple d'au moins 95 %. L'homologie peut être déterminée par comparaison visuelle ou au moyen de tout outil informatique généralement utilisé dans le domaine, tel que les programmes BLAST, disponibles sur www.ncbi.nlm.nih.gov, et utilisés avec les paramètres configurés par défaut.

L'homologie de séquence peut résulter de modifications issues de mutation ou de variation dans les séquences des peptides selon l'invention, provenant soit de la délétion ou de l'insertion d'un ou plusieurs acides aminés, soit de la substitution d'un ou plusieurs acides aminés dans les séquences caractéristiques d'un polypeptide selon l'invention.

On entend désigner par « fragment » toute portion peptidique, comprenant au moins 4, au moins 6, en particulier au moins 8, et plus particulièrement au moins 12 acides aminés consécutifs de la Desmogléine I, et une activité biologique sensiblement similaire. De manière générale, les analogues polypeptidiques peuvent comprendre des substitutions conservatrices par rapport à la séquence d'acides aminés naturelle.

Plusieurs de ces modifications peuvent être combinées.

A titre d'exemple de mutations que l'on peut considérer dans la présente description, il peut être mentionné, de manière non exhaustive, le remplacement d'un ou plusieurs résidus d'acides aminés par des résidus d'acides aminés ayant un indice hydropathique similaire sans pour autant affecter de manière sensible les propriétés biologiques native de la Desmogléine I.

L'indice hydropathique est un indice attribué aux acides aminés en fonction de leur hydrophobicité et de leur charge (Kyte et al. (1982), J. Mol. Biol., 157 : 105).

Les formes solubles également visées dans la présente description peuvent dériver de polypeptides ayant subi une ou plusieurs modification(s) post-traductionnelle(s).

Par « modification(s) post-traductionnelle(s) », on entend englober l'ensemble des modifications qu'un peptide ou une protéine est susceptible de subir à l'issue de sa synthèse dans une cellule, telle que par exemple une ou des phosphorylation(s), une ou des thiolation(s), une ou des acétylation(s), une ou des glycosylation(s), une ou des lipidation(s), telles qu'une farnésylation ou une palmitoylation, un réarrangement structural de type formation de ponts disulfures et/ou de type clivage à l'intérieur de la séquence peptidique.

L'analogue présente, par ailleurs, sensiblement la même activité biologique que le polypeptide naturel.

Selon un mode de réalisation, les formes solubles convenant à la mise enoeuvre de l'invention peuvent également être des polypeptides naturels ou synthétiques, obtenus après lyse enzymatique ou chimique de la forme native de la Desmogléine I ou par synthèse chimique ou biologique ou par extraction à partir d'un tissu biologique, comme par exemple la peau, exprimant naturellement ces formes solubles, ainsi que les différentes formes post-traductionnelles de celle-ci.

Selon un mode de réalisation, une forme soluble complexée conforme à l'invention peut dériver de l'association de la Desmogléine I ou de l'un de ses fragments avec soit une autre protéine Desmogléine I ou l'un de ses fragments, soit une protéine cible.

Une protéine de cible peut notamment être choisie parmi la Desmocolline 2a (Dsc2), les Plakophilines 1, 2 et 3, la Plakoglobine, la Kallikreine 5, l'hormone de croissance 1 (GH1), la SSSCA1 (Centromeric Autoantigen 27 kD), la RuvB-like 1, la protéine C3orf10, et la VRK3 (Vaccinia related kinase 3).

L'homme de l'art peut obtenir les formes solubles conformes à l'invention au moyen de procédés à base d'ADN recombinant, comme par exemple, ceux décrits dans le manuel « Molecular Cloning - A Laboratory Manual » (2ème édition), Sambrook et al., 1989, Vol. I-III, Coldspring Harbor Laboratory, Coldspring Harbor Press, NY, (Sambrook).

Selon un autre mode de réalisation, les formes solubles convenant à la mise en oeuvre de l'invention peuvent également se présenter sous une forme fusionnée avec un autre polypeptide distinct de ceux identifiés précédemment, un agent de ciblage hydrophile ou hydrophobe, un précurseur de bio-conversion, un agent de marquage luminescent, radioactif ou colorimétrique.

De manière non limitative, on peut citer comme exemple de composés susceptibles d'être couplés avec les formes solubles conformes à l'invention, des protéines fluorescentes comme la « Green Fluorescent Protein », des composés chimiques fluorescents tels que la rhodamine, la fluorescéine, ou le Texas Red^{®}, des composés phosphorescents, des éléments radioactifs, tels que ³H, ¹⁴C, ³⁵S, ¹²¹I ou ¹²⁵I, ou des agents de marquage colorimétrique comme les substrats chromogènes sensibles à l'action de la galactosidase, de la peroxydase, de la chloramphénicol acétyltransférase, de la luciférase ou de la phosphatase alcaline.

Selon la nature des composés susceptibles d'être couplés avec les formes solubles conformes à l'invention, le couplage peut être opéré par des procédés chimiques, notamment au moyen de fonctions chimiques réactives ou par des procédés de biologie moléculaire connus de l'homme de l'art.

A titre de méthodes de détection d'un polypeptide, il peut être fait mention du Western-blot, du Slot-blot, du Dot-blot, des méthodes ELISA (Enzyme Linked Immuno-Sorbent Assay) de type singleplex ou multiplex, des méthodes de protéomique ou de glycomique, de coloration de polypeptides dans un gel de polyacrylamide par un colorant à base d'Argent, par le bleu de Coomassie ou par le SYPRO, de l'immunofluorescence, de l'absorption UV, de méthodes immunohistochimiques en microscopie classique, électronique ou confocale, de FRET (fluorescence résonance energy transfer/transfert d'énergie par résonance de fluorescence), des méthodes de TR-FRET (time resolved FRET/FRET en résolution de temps), des méthodes de FLIM (fluorescence lifetime imaging microscopy/imagerie par microscopie uu temps de fluorescence), des méthodes de FSPIM (fluorescence spectral imaging microscopy/imagerie par microscopie en spectre de fluorescence), des méthodes de FRAP (fluorescence recovery after photobleaching/recouvrement de fluorescence après photoblanchiment), des méthodes par gène rapporteur, des méthodes d'AFM (atomic force microscopy/microscopie par force atomique), des méthodes de résonance plasmonique de surface, des méthodes de microcalorimétrie, des méthodes de cytométrie en flux, des méthodes de biosenseurs, des méthodes de radioimmuno-essais (RIA), des méthodes de focalisation isoélectrique, et des tests enzymatiques, des méthodes mettant en oeuvre des puces à peptides, des puces à sucre, des puces d'anticorps, des méthodes de spectrométrie de masse, des méthodes de spectrométrie de type SELDI-TOF (Ciphergen).

Comme il ressort de ce qui précède, les formes solubles considérées selon l'invention sont avantageusement caractérisées par une méthode ELISA et plus particulièrement par celle décrite dans l'exemple 1 ci-après.

### Utilisation des formes solubles selon l'invention, à des fins de criblage d'actifs anti-âge et/ou de caractérisation de l'efficacité d'un traitement contre les signes du vieillissement cutané.

Comme précisé précédemment, la présente description décrit selon un de ses aspects, des procédés non invasifs pour caractériser, notamment de façon *in vitro* ou *ex vivo*, l'efficacité d'un actif anti-âge ou d'un traitement cosmétique ou thérapeutique via l'analyse qualitativement ou quantitativement des formes solubles conformes à l'invention.

Ces procédés sont particulièrement avantageux dans la mesure où leur mise en oeuvre ne nécessite pas de recourir obligatoirement à une technique opératoire pour procéder à une telle caractérisation.

Les procédés selon l'invention, décrits ci-après, peuvent être effectués sur un échantillon, par exemple isolé, d'épithélium, et notamment d'épiderme, prélevé chez un individu.

Les procédés selon l'invention peuvent également être effectués sur un échantillon d'épithélium, et notamment d'épiderme, prélevé à partir d'un modèle cellulaire épithélial, et notamment épidermique, ou d'une peau isolée reconstruite afin d'en qualifier l'état.

Un extrait de l'épiderme peut ainsi être obtenu par simple stripping et directement analysé par la méthode ELISA telle que décrite dans l'exemple 1, ci-après.

La technique de stripping consiste à appliquer une surface collante à la surface de l'épiderme comme le Blenderm^{®} de 3M, le D'squam (adhésif commercial de CuDERM), la colle cyanoacrylate. Grâce à ces strippings, les cornéocytes adhérents et le contenu de leurs espaces intercellulaires peuvent être prélevés et soumis par la suite à une extraction permettant d'avoir accès au contenu protéique.

Le prélèvement d'un échantillon convenant au procédé peut aussi être effectué de façon plus directe par « lavage(s) » de la surface cutanée au moyen par exemple d'accessoires de type turbine à ailette, de type cellule à spirale (tel que décrit dans le brevet FR 2 667 778) associée à un circuit fluidique, ou simplement par ajout/prélèvement d'une goutte de tampon à la surface de la peau.

A titre indicatif, d'autres méthodes de prélèvement adaptées à la mise en oeuvre de l'invention, on peut mentionner des méthodes par grattage de la partie supérieure du *stratum corneum* au moyen d'un système bilames ou par shave biopsie. Cette technique permet de recueillir des squames qui peuvent ensuite être directement analysées par différentes techniques pour déterminer les taux en minéraux, aminoacides ou lipides.

A l'issue du prélèvement, l'échantillon est caractérisé par la méthode ELISA considérée en exemple 1, ci-après.

Cette méthode repose notamment sur la mise en oeuvre d'anticorps appropriés à la détection de formes solubles considérées selon l'invention.

Un anticorps susceptible d'être utilisé à titre d'outil d'évaluation d'un état d'un épiderme peut être obtenu par tout procédé connu de l'homme de l'art, tel que décrit dans « Antibodies: A Laboratory Manual », Cold Spring Harbor Laboratory Press, Cold Spring Harbor, N.Y. (1990). De façon avantageuse, les anticorps utilisés peuvent être des anticorps recombinants tels que ceux développés par la société Antibodies-by-design (AbD).

Telle que décrite dans l'exemple 1 ci-après, la méthode de détection des formes solubles de la Desmogléine I nécessite notamment l'utilisation de deux anticorps, à savoir un anticorps de capture développé avec la technologie HuCAL (AbD clone AbyD03984) et un anticorps de détection marqué au « sulfo-tag » selon les indications MSD (anticorps monoclonal, clone 129204, R&D systems).

### Criblage de composés biologiques ou chimiques.

Il est aussi décrit un procédé de criblage de composés biologiques ou chimiques, voire d'actifs anti-âge, ou de facteurs physico-chimiques, susceptibles d'agir au niveau de la Desmogléine I, et par conséquent d'influer sur la libération des formes solubles conformes à l'invention, comprenant au moins les étapes consistant à :
a) mettre en contact, avec au moins un composé chimique ou biologique à tester, au moins un type cellulaire apte à libérer au moins une forme peptidique soluble conforme à l'invention, dans des conditions propices à la libération de ladite forme peptidique soluble.
b) déterminer une teneur de ladite forme peptidique soluble, et
c) comparer ladite teneur déterminée à l'étape b) à une teneur de ladite forme soluble déterminée en absence de composé chimique ou biologique à tester.

Avantageusement, il peut être effectué, en outre, à l'issue de l'étape c), une étape de sélection du ou des actifs pour lequel ou lesquels on observe une augmentation de la teneur en forme(s) soluble(s).

La comparaison effectuée à l'étape c) peut permettre de déduire une information quant à la propriété dudit composé testé à modifier la Desmogléine I, et donc d'influer sur la libération des forme solubles conformes à l'invention.

A ce titre, un composé présentant une efficacité à l'égard de la Desmogléine I, qui s'accumule avec l'âge au niveau du *stratum corneum,* induit la libération de formes solubles de la Desmogléine I conformes à l'invention.

Une détection de la présence ou une augmentation de la teneur de ladite forme peptidique soluble est indicative d'un composé chimique ou biologique apte à agir au niveau de la Desmogléine I.

Plus particulièrement, le procédé utile pour caractériser l'efficacité d'un traitement susceptible de prévenir et/ou traiter les signes du vieillissement cutané chez un individu, peut comprendre au moins les étapes consistant à :
i. disposer d'au moins un premier échantillon de surface cutané représentatif dudit individu,
ii. quantifier au niveau dudit échantillon notamment *via* une technique de dosage ELISA au moins une forme soluble considérée dans la présente invention,
iii. renouveler les étapes i. et ii. sur un deuxième échantillon de surface cutané représentatif dudit individu, et
iv. comparer les résultats obtenus à l'issu des étapes ii. et iii., notamment afin d'en déduire une information relative à au moins un effet du traitement,
lesdits premier et second échantillons de surface cutané correspondant à des stades de traitement différents.

Dans l'hypothèse où une mesure de valeur de référence est effectuée préalablement à la mise en oeuvre du composé biologique ou chimique, voire d'un actif anti-âge, ou du facteur physico-chimique à tester, la méthode selon l'invention peut permettre en outre, le cas échéant, d'apprécier l'efficacité potentielle dudit composé.

La libération de forme(s) soluble(s) conforme(s) à l'invention peut ne pas être affectée par la présence dudit composé ou en revanche être stimulée.

Dans l'hypothèse où un effet stimulateur est constaté, le composé testé est susceptible d'être utilisé par exemple à titre d'actif anti-âge.

Un procédé conforme à l'invention peut être effectué sur un échantillon cellulaire isolé.

La détermination d'une teneur des formes solubles conformes à l'invention est effectuée au moyen de la méthode ELISA spécifique, telle que décrite en exemple 1, ci-après.

Le présent brevet décrit également l'utilisation d'une quantité efficace de formes solubles conformes à l'invention pour la préparation et/ou l'amélioration d'un modèle cellulaire pluristratifié, notamment de type épidermique ou muqueux, et en particulier un modèle de peau reconstruite.

Au sens de la description, on entend désigner par « modèle de peau reconstruite », un modèle dans lequel sont associés différents types cellulaires, tels que notamment les constituants naturels de la peau, à l'image par exemple des kératinocytes, des fibroblastes, des cellules de Langerhans et des mélanocytes.

Les cellules de type fibroblastes peuvent être irradiées ou non.

De tels modèles et leur préparation sont connus de l'homme de l'art.

Ainsi, le présent brevet décrit également un procédé de préparation d'une peau reconstruite isolée, comprenant au moins l'étape consistant à mettre en contact au moins une forme soluble conforme à l'invention avec des cellules susceptibles de générer une peau reconstruite isolée, et notamment des kératinocytes.

Au sens de la présente description, selon un autre de ses aspects, les formes solubles de la Desmogléine I telles que décrites précédemment peuvent être utilisée dans une composition cosmétiques ou thérapeutiques.

Il est entendu que l'ensemble des compositions cosmétiques ou thérapeutiques considérées selon l'invention, mettent en oeuvre un milieu physiologiquement acceptable.

Au sens de la présente invention, on entend désigner par «milieu physiologiquement acceptable», un milieu convenant à l'application d'une composition sur un épithélium ou une matière kératinique, telle que la peau, le cuir chevelu, les lèvres, les muqueuses et les fibres kératiniques comme les cheveux, les ongles et les poils, ou le cas échéant par voie orale ou parentérale.

Par exemple, il peut être décrit une composition cosmétique ou thérapeutique.

Une composition conforme à la description peut comprendre, en outre de la (ou des) forme(s) soluble(s), au moins un agent actif cosmétique et/ou thérapeutique.

Comme exemples d'agents actifs utilisables dans le cadre de la présente description, il peut être fait mention d'huiles cosmétiques, telles que les huiles de silicone, les huiles végétales de type triglycérides, les huiles hydrocarbonées telles que l'huile de Parleam et les esters d'acides gras et d'alcool gras.

D'autres agents actifs permettant d'améliorer l'état de la peau peuvent également être utilisé, tels que des actifs hydratants ou des agents actifs permettant d'améliorer la barrière lipidique naturelle, tels que des céramides, des sulfates de cholestérol et/ou des acides gras, et leurs mélanges.

Des enzymes ayant une activité sur la peau peuvent également être utilisées, telles que des protéases, des lipases, des glucosidases, des amidases, des cérébrosidases et/ou des mélanases, et leurs mélanges.

D'une manière générale, toute composition conforme à l'invention peut être appliquée sur la peau (sur toute zone cutanée du corps) ou sur les muqueuses (buccale, jugale, gingivale, génitale, conjonctivale, ...).

De façon connue, une composition cosmétique peut contenir également des adjuvants habituels dans le domaine cosmétique, tels que des gélifiants hydrophiles ou lipophiles, des additifs hydrophiles ou lipophiles, des conservateurs, des antioxydants, des solvants, des parfums, des charges, des filtres, des absorbeurs d'odeurs et des matières colorantes.

Les quantités des différents constituants des compositions décrites sont celles classiquement utilisées dans les domaines considérés.

Selon un autre aspect, la présente invention décrit un procédé de traitement cosmétique des signes de vieillissement cutané comprenant au moins une étape consistant à appliquer sur une partie au moins de la peau, des muqueuses et/ou des fibres kératiniques, au moins une composition cosmétique conforme à l'invention.

Selon un autre aspect, l'invention décrit également l'utilisation, notamment cosmétique et/ou thérapeutique, de formes peptidiques solubles de la Desmogléine I, ou d'analogues de celle-ci, ou d'un agent modulateur de l'activité, de l'expression et/ou de la stabilité d'un tel polypeptide, notamment pour prévenir les signes de vieillissement cutané, notamment pour prévenir et/ou traiter les peaux âgées.

L'invention décrit l'utilisation d'un agent modulateur de des formes solubles conformes à l'invention.

Au sens de la description, on entend par « moduler », au regard d'un effet donné, l'action de stimuler ou d'inhiber cet effet.

Au sens de la présente description, on entend par l'expression « agent modulateur ou composé chimique ou biologique apte à moduler l'activité biologique et/ou l'expression » tout composé apte à agir, directement ou indirectement, sur les formes solubles conformes à l'invention, ou une séquence d'acides nucléiques codant en partie pour celles-ci, ou sur un élément d'une voie de signalisation intra ou extra-cellulaire, ou d'une voie métabolique, impliquant lesdites formes solubles, ou sur un élément impliqué dans la régulation de la transcription et/ou de la traduction d'une séquence d'acides nucléiques codant pour lesdites formes solubles, ainsi que dans la régulation de la stabilité de celles-ci.

Cet agent modulateur peut être un agent inhibiteur ou activateur de l'expression des formes solubles de l'invention, ou encore un agent régulant la stabilité desdites formes solubles.

Plus particulièrement, l'agent modulateur peut être un activateur de l'expression des formes solubles selon l'invention.

Par exemple, l'agent modulateur est un agent favorisant la stabilité des formes solubles conformes à l'invention, en inhibant leur dégradation protéolytique.

Les exemples figurant ci-après sont présentés à titre illustratif et non limitatif de l'invention.
**Figure 1** **:** elle illustre l'efficacité du test ELISA haute sensibilité conforme à l'invention dans le suivi d'actifs agissant sur la dégradation de la Desmogléine I.
**Figure 2** **:** elle illustre les effets d'un un sérum composé à 10 % de Bifidiobiotic (complexe CLR) et 0,002 % de Phytosphingosine-SLC (traitement « G ») sur la quantité en formes peptidiques solubles conformes à l'invention.

### Exemple 1 :

### Mise au point d'un test ELISA haute sensibilité permettant le dosage de formes solubles de la Desmogléine I conformes à l'invention :

Des plaques 96 puits spécifiques « Desmogleine » ont été développées à façon avec la technologie de la société MesoScale Discovery (MSD).

L'anticorps recombinant de capture (clone AbyD03984), dirigé contre un domaine extracellulaire de la Desmogleine a été développé avec la technologie HuCAL (Société : Antibodies by Design) par un crible effectué avec une Desmogléine recombinante (recombinante R&D systems).

L'anticorps de capture a été déposé à une concentration de 200 µg/ml sur une plaque standard 96 puits de type « small spot » (MesoScale).

Une courbe de référence (400 à 6 ng/ml) a été établie utilisant une protéine recombinante Desmogléine. Chaque plaque a été bloquée par un tampon de blocage (MesoScale) pendant 1 heure à température ambiante. 25 µl de chaque échantillon et de standards ont été déposés en triple et incubées sous agitation pendant 1 heure à température ambiante. La plaque a été alors lavée 4 fois au tampon Tris (MesoScale). Les plaques ont été alors incubées avec agitation pendant 1 heure à température ambiante avec 25 µl d'anticorps de détection (anticorps monoclonal, clone 129204, R&D Systems) marqué au « sulfo-tag » selon les indications MSD à une concentration de 1 ug/ml. Les plaques ont été lavées 4 fois avec un tampon de Tris (MesoScale) avant l'addition de 150 µl de 1 X read buffer T (MesoScale). Les plaques ont été lues à l'aide du « Sector Imager 6000 ». Les données ont été standardisées en termes de ng de Desmogleine par µg de protéine totale mesurées par un kit Bradford (Bio-Rad).

### Exemple 2 :

### Utilisation du test ELISA haute sensibilité pour le criblage d'actifs agissant sur la dégradation de la Desmogléine 1.

### Protocole :

6 mg de *Stratum Corneum* Plantaire (SCP) finement broyé sont distribués dans chaque puits d'une plaque 96 puits Multiscreen filtration system 0.22 µm (Millipore). Chaque essai est tripliqué.

100 µl d'EDTA à 0.2 et 2 % en tampon Tris 0.1M pH8 sont ajoutés au SCP. La plaque est incubée 10 min à température ambiante. La plaque est ensuite filtrée, les puits sont rincés 5 fois avec 200 µl de tampon PBS. 200 µl de tampon Tris 0.1M pH8 sont ajoutés à chaque puits. La plaque est incubée 2h sous agitation à 30 °C puis filtrée dans une plaque de réception. Chaque milieu réactionnel est ainsi recueilli.

Le dosage des peptides de la Desmogléine 1 est réalisé sur plaque Mesoscale MA6000 DB016 dont le développement a été décrit précédemment.

Chaque dosage est réalisé directement avec 25 µl de milieu réactionnel. Une gamme d'étalonnage est réalisée dans les mêmes conditions avec la Desmogléine recombinante (R&D system). Les résultats peuvent être exprimés soit en UA (unités arbitraires = signal obtenu sur le « sector imager 6000 » ou en ng de Desmogléine 1 par µg de protéines totales).

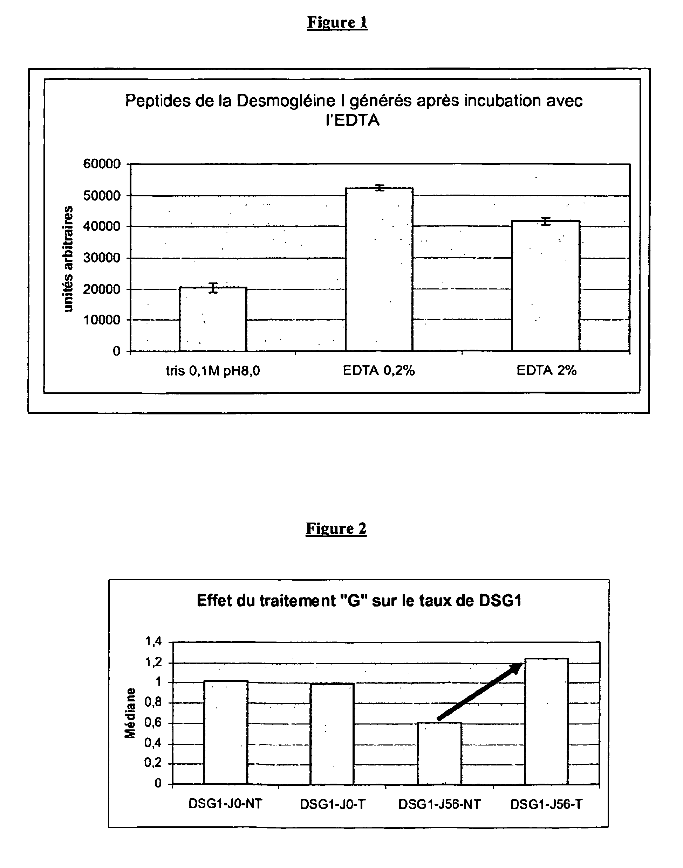

La figure 1 permet effectivement de suivre la libération de formes solubles de la Desmogléine 1 induite par un traitement favorisant la dégradation du cornéodesmosome, tel qu'un traitement par des chélateurs de la famille de l'EDTA.

Le test est donc adapté à l'évaluation d'un effet stimulant de dégradation de la Desmogléine 1 par mesure des formes solubles libérées in-vitro.

Ce test est donc indiqué pour suivre l'effet de molécules ou de formules cosmétiques, en particulier d'actifs anti-âges, permettant de compenser l'accumulation anormale de Desmogléine 1 observée dans le *stratum corneum* âgé.

### Exemple 3 :

### Caractérisation de la teneur en formes solubles de la Desmogléine I dans différents échantillons prélevés par stripping.

Cette caractérisation est réalisée à l'aide du test ELISA décrit en exemple 1.

Une étude produit a été réalisée sur 56 jours, soit 2 mois (1 groupe de 24 sujets femmes, peau caucasienne, âgées de 40-45 ans) avec des prélèvements effectués sur l'avant-bras.

Le produit est un sérum composé à 10 % de Bifidiobiotic (complexe CLR) et 0,002 % de Phytosphingosine-SLC (traitement « G »).

Le complexe CLR se rapporte à un lysat enregistré sous le nom INCI : Bifidat ferment Lysate, sous le nom EINECS : Bifidobacterium longum, sous le N° EINECS : N° 306-168-4 et sous le N° CAS : N° 96507-89-0.

Un tel lysat est notamment commercialisé sous la dénomination Repair Complex CLR^{®} par la société K. RICHTER GmbH. Le dérivé phytosphingosine-alicylate est celui commercialisé par la société EVONICK GOLDSCHMIDT sous la dénomination Phytosphingosine SLC.

Pour cette étude, des prélèvements D-Squame ont été fait au niveau de l'avant-bras (face postérieure).

### a) Extraction des protéines des cornéodiscs D-Squame :

Le cornéodisc est placé dans un tube Eppendorf 2 ml, face collante vers l'intérieur, auquel on ajoute 550 µl de tampon d'extraction dit « Native+ » (TBS, 1 % Triton X-100, 1M NaCl) et une bille en acier inox par tube. Le tube est ensuite placé dans les portoirs du vibrobroyeur MM400 (Retsch). L'extraction se fait par vibrobroyage pendant un cycle de 2 min à 30 Htz. Le milieu est ensuite récupéré et filtré sur colonne Millipore Ultrafree de 0.45 µm suivi d'une centrifugation à 5000 g à 4 °C pendant 5 min. Le surnageant est conservé à -20 °C en attendant d'être analysé.

### b) Analyse statistique

L'analyse, pour l'effet traitement, est réalisée à l'aide d'un modèle mixte d'analyse de la variance sur les différences appariées Actif - Placebo avec le facteur temps, en effet fixe, et le facteur sujet en effet aléatoire.

L'effet traitement à J0 et J56 est testé à l'aide de contrastes.

Les variables sont, lorsque c'est nécessaire, préalablement log transformées pour rendre leur distribution plus Gaussienne.

On utilise le logiciel SPSS version 14 pour les analyses descriptives (graphiques des moyennes et box-plots), et le logiciel SAS Enterprise Guide version 3 pour la partie interférentielle.

Le risque alpha de première espèce est fixé à 5 % dans une approche bilatérale.

Les résultats de cette étude sont illustrés par la figure 2.

La figure 2 révèle l'effet du produit testé sur la quantité en formes solubles. On constate une augmentation significative de la libération de formes solubles de la Desmogléine I, ce qui démontre l'effet stimulateur de ce produit sur la libération de la Desmogléine I.

Le produit testé présente donc un réel effet anti-âge puisqu'il permet de contrer l'accumulation en Desmogléine **I** avec l'âge.

## Revendications

1. Procédé *in vitro* ou *ex vivo* de criblage d'actifs anti-âge comprenant au moins les étapes consistant à :
a) mettre en contact, au moins un type cellulaire apte à libérer au moins une forme peptidique soluble complexée ou non, dérivant de la protéolyse du polypeptide de séquence d'acides aminés représentée en SEQ ID NO 2, avec au moins un actif anti-âge à tester, dans des conditions propices à une libération de ladite forme peptidique soluble,
b) déterminer une teneur en ladite forme peptidique soluble, et
c) comparer ladite teneur déterminée à l'étape b) à une teneur de ladite forme soluble déterminée en absence de composé chimique ou biologique à tester,
dans lequel une détection ou une caractérisation d'une présence ou d'une augmentation d'une teneur de ladite forme peptidique soluble est significative d'une efficacité dudit actif.

2. Procédé *in vitro* ou *ex vivo* pour caractériser l'efficacité d'un traitement cosmétique ou thérapeutique visant, chez un individu, à prévenir et/ou traiter les signes de vieillissement cutané liés au vieillissement chronologique, comprenant au moins la caractérisation qualitative ou quantitative d'au moins une forme peptidique soluble complexée ou non, dérivant de la protéolyse du polypeptide de séquence d'acides aminés représentée en SEQ ID NO 2, dans lequel une détection ou une caractérisation d'une présence ou d'une augmentation d'une teneur de ladite forme peptidique soluble est significative d'une efficacité dudit traitement.

3. Procédé selon la revendication 2, comprenant au moins les étapes consistant à :
i. disposer d'au moins un premier échantillon de surface cutané représentatif dudit individu,
ii. quantifier au niveau dudit échantillon notamment via une technique de dosage ELISA, au moins une forme peptidique soluble complexée ou non, dérivant au moins en partie de la protéolyse du polypeptide de séquence d'acides aminés représentée en SEQ ID NO 2,
iii. renouveler les étapes i. et ii. sur un deuxième échantillon de surface cutané représentatif dudit individu, et
iv. comparer les résultats obtenus à l'issu des étapes ii. et iii., notamment afin d'en déduire une information relative à au moins un effet dudit traitement,
dans lequel la détection à l'étape iv. d'une présence ou d'une augmentation d'une teneur de ladite forme peptidique soluble est indicative d'une efficacité dudit traitement, et
dans lequel lesdits premier et second échantillons de surface cutané correspondant à des stades de traitement différents.

4. Procédé selon la revendication précédente, dans lequel le premier échantillon est représentatif d'un état pré-traitement et le second est représentatif d'un état en cours de traitement ou post-traitement.

5. Procédé selon la revendication 4, comprenant, en outre, une étape consistant à ajuster chez ledit individu ledit traitement par administration d'une composition cosmétique de soin établie ou sélectionnée au regard de l'information obtenue à l'issue de l'étape iv.

6. Procédé selon l'une des revendications 1 à 5, dans lequel ladite forme soluble complexée dérive de l'association de la Desmogléine 1 ou de l'un de ses fragments avec soit une autre protéine Desmogléine 1 ou l'un de ses fragments, soit une protéine cible, notamment choisie parmi la Desmocolline 2a (Dsc2), les Plakophilines 1, 2 et 3, la Plakoglobine, la Kallikreine 5, l'hormone de croissance 1 (GH1), la SSSCA1 (Centromeric Autoantigen 27 kD), la RuvB-like 1, la protéine C3orf10, et la VRK3 (Vaccinia related kinase 3).

## Patentansprüche

1. *In vitro* oder *ex vivo* Sreeeningverfahren auf Anti-aging-Wirkstoffe, umfassend mindestens die folgenden Schritte:
a) Inkontaktbringen von mindestens einem Zelltyp, der dazu ausgelegt ist, mindestens eine lösliche komplexierte oder nicht komplexierte Peptidform freizusetzen, die sich aus der Proteolyse des Polypeptids mit der durch SEQ ID NO: 2 dargestellten Aminosäuresequenz ableitet, mit mindestens einem zu testenden Anti-aging-Wirkstoff unter Bedingungen, die für eine Freisetzung der löslichen Peptidform günstig sind,
b) Bestimmen eines Gehalts der löslichen Peptidform, und
c) Vergleichen des in Schritt b) bestimmten Gehalts mit einem Gehalt der in Abwesenheit von zu testender chemischer oder biologischer Verbindung bestimmten löslichen Form,
wobei ein Nachweis oder eine Charakterisierung einer Gegenwart oder eines Anstiegs eines Gehalts der löslichen Peptidform ein Hinweis auf die Wirksamkeit des Wirkstoffs ist.

2. *In vitro* oder *ex vivo* Verfahren zur Charakterisierung der Wirksamkeit einer kosmetischen oder therapeutischen Behandlung, die darauf ausgerichtet ist, bei einem Individuum die Anzeichen von mit der Zeitalterung verbundenen Anzeichen der Hautalterung zu verhindern oder zu behandeln, umfassend mindestens die qualitative oder quantitative Charakterisierung von mindestens einer löslichen komplexierten oder nicht komplexierte Peptidform, die sich aus der Proteolyse des Polypeptids mit der durch SEQ ID NO: 2 dargestellten Aminosäuresequenz ableitet, wobei ein Nachweis oder eine Charakterisierung einer Gegenwart oder eines Anstiegs eines Gehalts der löslichen Peptidform ein Hinweis auf eine Wirksamkeit der Behandlung ist.

3. Verfahren nach Anspruch 2, umfassend mindestens die Schritte bestehend aus:
i) Bereitstellen von mindestens einer ersten Probe von Hautoberfläche, die für das Individuum repräsentativ ist,
ii) Quantifizieren in der Probe, insbesondere über eine ELISA-Testtechnik, von mindestens einer löslichen komplexierten oder nicht komplexierten Peptidform, die sich mindestens teilweise aus der Proteolyse des Polypeptids mit der durch SEQ ID NO: 2 dargestellten Aminosäuresequenz ableitet,
iii) Wiederholen der Schritte i. und ii. mit einer zweiten Probe von Hautoberfläche, die für das Individuum repräsentativ ist, und
iv) Vergleichen der in den Schritten ii. und iii. erhaltenen Ergebnisse, insbesondere um daraus eine Information bezüglich mindestens einer Wirkung der Behandlung abzuleiten,
wobei der Nachweis in Schritt iv. einer Gegenwart oder eines Anstiegs eines Gehalts der löslichen Peptidform ein Hinweis auf die Wirksamkeit der Behandlung ist, und wobei die ersten und zweiten Proben von Hautoberfläche verschiedenen Behandlungsstadien entsprechen.

4. Verfahren nach dem vorhergehenden Anspruch, wobei die erste Probe repräsentativ für einen Vorbehandlungszustand ist und die zweite Probe repräsentativ für einen Zustand im Laufe der Behandlung oder Nachbehandlung ist.

5. Verfahren nach Anspruch 4, umfassend weiterhin einen Schritt bestehend aus dem Einstellen der Behandlung bei dem Individuum durch Verabreichung einer kosmetischen Pflegezusammensetzung, die im Hinblick auf die durch Schritt iv erhaltene Information entwickelt oder ausgewählt wurde.

6. Verfahren nach einem der Ansprüche 1 bis 5, wobei sich die lösliche komplexierte Form aus der Assoziation von Desmoglein I oder einem seiner Fragmente mit entweder einem anderen Desmoglein I-Protein oder einem seiner Fragmente oder einem Zielprotein ableitet, das insbesondere aus Desmocollin 2a (Dsc2), Plakophilinen 1, 2, und 3, Plakoglobin, Kallikrein 5, Wachstumshormon 1 (GH1), SSSCA1 (zentromeres Autoantigen 27 kD), RuvB-like 1, C3orf10-Protein und VRK3 (Vaccinia verwandte Kinase 3) ausgewählt ist.

## Claims

1. *In vitro* or *ex vivo* method to screen anti-ageing active agents, comprising at least the steps of:
a) contacting at least one cell type capable of releasing at least one soluble peptide form whether or not complexed, derived from proteolysis of the polypeptide having the amino acid sequence represented by SEQ ID NO 2, with at least one test anti-ageing active agent, under conditions suitable for release of said soluble peptide form;
b) determining a content of said soluble peptide form; and
c) comparing the said content determined at step b) with a content of the said soluble form determined in the absence of chemical or biological compound to be tested,
wherein the detection or characterization of the presence or an increase in a content of the said soluble peptide form is indicative of an efficacy of the said agent.

2. *In vitro* or *ex vivo* method to characterize the efficacy of a cosmetic or therapeutic treatment intended, in an individual, to prevent and/or treat the signs of skin ageing linked to chronological ageing, comprising at least the qualitative or quantitative characterization of at least one soluble peptide form, whether or not complexed, derived from proteolysis of the polypeptide having the amino acid sequence represented by SEQ ID NO 2, wherein the detection or characterization of the presence of an increase in a content of the said soluble peptide form is indicating an efficacy of the said treatment.

3. The method according to claim 2 comprising at least the steps of
i. providing at least a first skin surface sample representative of said individual;
ii. quantifying, in said sample, in particular via an ELISA assay technique, at least one soluble peptide form, whether or not complexed, derived at least in part from proteolysis of the polypeptide having the amino acid sequence represented by SEQ ID NO 2;
iii. repeating steps i. and ii. on a second skin surface sample representative of said individual; and
iv. comparing the results obtained at the end of steps ii. and iii., in particular so as to infer therefrom information relating to at least one effect of the said treatment,
wherein the detection at step iv) to detect the presence or an increase in the content of the said soluble peptide form is indicative of an efficacy of the said treatment; and
wherein the said first and second samples of skin surface correspond to different stages of treatment.

4. The method according to the preceding claim wherein the first sample is representative of a pre-treatment state and the second is representative of a state in the progress of treatment or post-treatment.

5. The method according to claim 4 further comprising a step to adjust said treatment in the said individual by administering a cosmetic care composition that is proven or selected having regard to the information obtained after step iv.

6. The method according to one of claims 1 to 5 wherein the said complexed soluble form derives from the association of Desmoglein-I or one of the fragments thereof either with another Desmoglein-1 protein or one of the fragments thereof, or with a target protein selected in particular from among Desmocollin 2a (Dsc2), Plakophilins 1, 2 and 3, Plakoglobin, Kallikrein 5, growth hormone 1 (GH1), SSCA1 (Centromeric Autoantigen 27 kD), RuvB-like1, C3orf10 protein and VRK3 ( Vaccinia related kinase 3).


### SEQUENCE LISTING

<110> L'OREAL
<120> Utilisation à des fins de criblage d'actifs anti-âges de formes solubles
   de la protéine de type Desmogléine I
<130> BR87469/96082/KLP/PLC/cf
<160> 85
<170> PatentIn version 3.3
<210> 1
   <211> 4512
   <212> DNA
   <213> Homo Sapiens
<400> 1
   cccagcccaa gtttttaggg tggggatcca gactggttat acgtaccttc agtccttctc     60
   ccagaggaag gcagaaacac ctcaaagcct gcatgtaaga acatctactg agaaattatt    120
   ttaatcagac accagctgag tgggagaaag gaaaagaaca gagaagaaca aacaaaactc    180
   ccttggtctt ggatgtaaga gaatccagca gagatggact ggagtttctt cagagtagtt    240
   gcagtgctgt tcatttttct ggtggtggta gaagttaaca gtgaattccg aatccaggta    300
   agagattata acactaaaaa tggcaccatc aaatggcatt caatccgaag gcagaaacgt    360
   gaatggatca agttcgcagc agcctgtcgt gaaggtgaag acaactcaaa gaggaaccca    420
   atcgccaaaa ttcactcaga ttgtgctgca aaccagcaag ttacataccg catctctgga    480
   gtaggaattg atcagccacc atatgggatc tttgtcatta atcagaaaac tggtgaaatt    540
   aatataacat ccatagttga tcgagaggtc actcctttct tcattatcta ctgccgagct    600
   ctgaactcaa tgggccaaga tttagagagg cctctagagc tcagagtcag ggttttggat    660
   ataaatgaca accctccagt gttttcaatg gctacatttg caggacaaat agaagaaaat    720
   tctaatgcaa atacactggt gatgatactc aatgctactg acgcagatga accgaacaat    780
   ttgaactcaa aaatagcctt caagattata agacaagaac cttcagattc accaatgttt    840
   attatcaaca gaaatactgg agaaattcga acgatgaata attttctaga cagagagcaa    900
   tacggccagt atgctcttgc tgtaagaggc tctgaccgag atggtggggc agatggcatg    960
   tcagcggaat gtgagtgcaa cattaaaatc ctcgatgtca atgataatat cccttacatg   1020
   gaacagtctt catataccat agaaattcaa gaaaatactc taaattcaaa tttgctcgag   1080
   attagagtaa ttgatttgga tgaagagttc tcagctaact ggatggcagt aattttcttt   1140
   atctctggaa atgaaggaaa ttggtttgag atagaaatga atgaaagaac aaatgtggga   1200
   attttaaagg ttgttaagcc cttagattat gaagctatgc agagtctgca actcagtatt   1260
   ggtgtcagaa ataaagctga atttcatcat tcaattatgt ctcaatataa actgaaagca   1320
   tctgcaattt ctgtgactgt gttaaatgta attgaaggcc cagtgtttcg tccaggttca   1380
   aagacatatg ttgtaactgg taatatggga tcaaatgata aagtgggaga ctttgtagct   1440
   actgacctgg acacaggtag accttcaacg actgttaggt atgtaatggg aaataatcca   1500
   gctgacctgc tagctgttga ttcaagaaca ggcaaactca ctttgaaaaa taaagttacc   1560
   aaggaacagt acaatatgct cggaggaaaa taccaaggaa cgattctctc tatagatgat   1620
   aatcttcaaa gaacttgcac tggtacaatt aatattaaca ttcaaagttt tggtaatgac   1680
   gacaggacta atacagagcc gaacactaaa attactacca atactggcag acaagaaagt   1740
   acttcttcca ctaactatga taccagcaca acttctactg actctagcca agtatattct   1800
   tctgaacccg gaaacggagc caaagatttg ttatcagaca atgtacattt tggtcctgct   1860
   ggcattggac tcctcatcat gggattcttg gtcttaggat tggtcccatt tttgatgatc   1920
   tgttgtgatt gtggaggtgc tcctcgtagt gcagctggct ttgagcctgt tcccgaatgt   1980
   tcagatggag caattcattc atgggcagta gaaggaccac agcctgaacc cagggatata   2040
   accactgtca taccacaaat accacctgat aacgcaaata taattgaatg cattgacaac   2100
   tcaggagttt atacaaatga gtatggtggc agagaaatgc aagatctggg aggaggagag   2160
   agaatgacag gatttgaact aacagaggga gttaaaactt caggaatgcc tgagatatgt   2220
   caagaatact ctggaacatt aagaagaaat tctatgaggg aatgtagaga aggaggtctg   2280
   aatatgaatt tcatggaaag ctacttctgt cagaaagcat atgcttacgc agatgaagat   2340
   gaaggacgcc catctaatga ctgtttgctc atatatgaca tcgaaggtgt aggttcccct   2400
   gctggctctg tgggttgttg tagcttcatt ggagaagacc tggatgacag cttcttggat   2460
   accctgggac ctaaatttaa gaagttggca gacatcagcc taggaaaaga atcatatcca   2520
   gaccttgatc cttcttggcc accacaaagc actgaaccag tttgccttcc tcaggaaaca   2580
   gagcccgttg ttagtggaca cccaccaatc tccccacatt tcggcactac cacagtaatt   2640
   tctgagagca cctatccctc gggacctggt gtactgcatc ctaagcctat tctcgatcct   2700
   ctgggctatg gtaatgtcac tgtgaccgag tcttacacca cctctgacac tctgaagccc   2760
   tctgtgcacg ttcacgataa ccgaccagca tcaaacgtgg tagtgacaga gagagtggtc   2820
   ggcccaatct ctggcgctga tttgcatgga atgttagaga tgcctgactt gcgagatggg   2880
   tcgaatgtta tagtgacaga aagggtaata gcaccaagct ctagtctacc cacctctctg   2940
   actatccatc atcctagaga gtcttcaaat gtggtagtga cagaaagagt aatccaacca   3000
   acttccggca tgataggtag tctgagtatg caccccgagt tagccaatgc ccacaatgtc   3060
   attgtgacag agagggttgt ttctggtgct ggcgtaactg gaattagtgg caccactggg   3120
   atcagcggtg gcataggcag cagtggcctg gttggcacca gcatgggtgc tgggagcggt   3180
   gccctgagtg gagctggcat aagtggtggt ggcattggcc tgagcagctt gggagggaca   3240
   gccagcattg gccacatgag gagttcctct gaccatcact ttaaccaaac cattgggtcc   3300
   gcctccccta gcacagctcg aagtcgaatc acaaagtata gtaccgtgca atatagcaag   3360
   tagtcaggac cccagctcac tttttcatag tcattgtggt ttagatccaa ttcccaccac   3420
   taaaaaacta acaatgtgat ttataacgca caacttcgtg ctcaggtcat ctaggagcaa   3480
   ggtgagaaat cacaatgaga aaaataaatg gaaacaccac tgctagggga gagctctcct   3540
   tagcattcat aaacttttct cttatattag gactaaggaa ctaaaacttg aggcagagtc   3600
   ttctttgtgc ctgagtggcc tgtagtccat ctccagcatg taactggcct tacgatggca   3660
   attggcatca ttctccttgc tctgttttgc ttttccatat agctcgagca aaattcaaaa   3720
   agaactaaat atgcaatata tgttcatatc tatgggaaaa atctaaaatg tgtgccagat   3780
   gccctgttgg tttcacagat aacataaata aaaattcaac cacagattta tacaagggtt   3840
   aaccattttt tttaagtttg actacatagt caagtccaca agccatcaag cactcctacc   3900
   ttaattattg cactagagaa aataaattcc aaattaggaa gtgtttccta ggaggaaaat   3960
   tccattagag agtggcaata ggatgaggtt tcttcagggt aaactagcaa tgcctgagcc   4020
   tgaaccttaa tgtggggcct cagttaatat ccctgtggag tcaaggattc ttctgattct   4080
   agtgtgtgtt tagtgataga tgtagtcttg acgaatattg cttactggtg aggttgagga   4140
   atatcacact cgtctttccc tttaccactg tggttttgac ttaagaaagc aaaactcact   4200
   aagtttactt ctcgaattga agcaagtgag gcctgacatg gttgtcatca ctagtggcaa   4260
   atgaccttcc aagtaagcag atgggaactg aattgtgttt tcaggttttg tttttagtag   4320
   gtgatattca ttcgtatcca gctctttatt acatagctct gaagttaaaa tgatttacat   4380
   aggccgagct gtggacaaaa aaaaagaagc agcagcttgt agtatgctta agctttgggg   4440
   aatttttttt taaggggatc taaaaaaatg tttttagaac atgtaaaatg tttaatggtg   4500
   aaagttggaa aa                                                       4512
<210> 2
   <211> 1049
   <212> PRT
   <213> Homo Sapiens
<400> 2
   Met Asp Trp Ser Phe Phe Arg Val Val Ala Val Leu Phe Ile Phe Leu
   1               5                   10                  15
   Val Val Val Glu Val Asn Ser Glu Phe Arg Ile Gln Val Arg Asp Tyr
               20                  25                  30
   Asn Thr Lys Asn Gly Thr Ile Lys Trp His Ser Ile Arg Arg Gln Lys
           35                  40                  45
   Arg Glu Trp Ile Lys Phe Ala Ala Ala Cys Arg Glu Gly Glu Asp Asn
       50                  55                  60
   Ser Lys Arg Asn Pro Ile Ala Lys Ile His Ser Asp Cys Ala Ala Asn
   65                  70                  75                  80
   Gln Gln Val Thr Tyr Arg Ile Ser Gly Val Gly Ile Asp Gln Pro Pro
                   85                  90                  95
   Tyr Gly Ile Phe Val Ile Asn Gln Lys Thr Gly Glu Ile Asn Ile Thr
               100                 105                 110
   Ser Ile Val Asp Arg Glu Val Thr Pro Phe Phe Ile Ile Tyr Cys Arg
           115                 120                 125
   Ala Leu Asn Ser Met Gly Gln Asp Leu Glu Arg Pro Leu Glu Leu Arg
       130                 135                 140
   Val Arg Val Leu Asp Ile Asn Asp Asn Pro Pro Val Phe Ser Met Ala
   145                 150                 155                 160
   Thr Phe Ala Gly Gln Ile Glu Glu Asn Ser Asn Ala Asn Thr Leu Val
                   165                 170                 175
   Met Ile Leu Asn Ala Thr Asp Ala Asp Glu Pro Asn Asn Leu Asn Ser
               180                 185                 190
   Lys Ile Ala Phe Lys Ile Ile Arg Gln Glu Pro Ser Asp Ser Pro Met
           195                 200                 205
   Phe Ile Ile Asn Arg Asn Thr Gly Glu Ile Arg Thr Met Asn Asn Phe
       210                 215                 220
   Leu Asp Arg Glu Gln Tyr Gly Gln Tyr Ala Leu Ala Val Arg Gly Ser
   225                 230                 235                 240
   Asp Arg Asp Gly Gly Ala Asp Gly Met Ser Ala Glu Cys Glu Cys Asn
                   245                 250                 255
   Ile Lys Ile Leu Asp Val Asn Asp Asn Ile Pro Tyr Met Glu Gln Ser
               260                 265                 270
   Ser Tyr Thr Ile Glu Ile Gln Glu Asn Thr Leu Asn Ser Asn Leu Leu
           275                 280                 285
   Glu Ile Arg Val Ile Asp Leu Asp Glu Glu Phe Ser Ala Asn Trp Met
       290                 295                 300
   Ala Val Ile Phe Phe Ile Ser Gly Asn Glu Gly Asn Trp Phe Glu Ile
   305                 310                 315                 320
   Glu Met Asn Glu Arg Thr Asn Val Gly Ile Leu Lys Val Val Lys Pro
                   325                 330                 335
   Leu Asp Tyr Glu Ala Met Gln Ser Leu Gln Leu Ser Ile Gly Val Arg
               340                 345                 350
   Asn Lys Ala Glu Phe His His Ser Ile Met Ser Gln Tyr Lys Leu Lys
           355                 360                 365
   Ala Ser Ala Ile Ser Val Thr Val Leu Asn Val Ile Glu Gly Pro Val
       370                 375                 380
   Phe Arg Pro Gly Ser Lys Thr Tyr Val Val Thr Gly Asn Met Gly Ser
   385 390 395 400
   Asn Asp Lys Val Gly Asp Phe Val Ala Thr Asp Leu Asp Thr Gly Arg
   405 410 415
   Pro Ser Thr Thr Val Arg Tyr Val Met Gly Asn Asn Pro Ala Asp Leu
   420 425 430
   Leu Ala Val Asp Ser Arg Thr Gly Lys Leu Thr Leu Lys Asn Lys Val
   435 440 445
   Thr Lys Glu Gln Tyr Asn Met Leu Gly Gly Lys Tyr Gln Gly Thr Ile
   450 455 460
   Leu Ser Ile Asp Asp Asn Leu Gln Arg Thr Cys Thr Gly Thr Ile Asn
   465 470 475 480
   Ile Asn Ile Gln Ser Phe Gly Asn Asp Asp Arg Thr Asn Thr Glu Pro
   485 490 495
   Asn Thr Lys Ile Thr Thr Asn Thr Gly Arg Gln Glu Ser Thr Ser Ser
   500 505 510
   Thr Asn Tyr Asp Thr Ser Thr Thr Ser Thr Asp Ser Ser Gln Val Tyr
   515 520 525
   Ser Ser Glu Pro Gly Asn Gly Ala Lys Asp Leu Leu Ser Asp Asn Val
   530 535 540
   His Phe Gly Pro Ala Gly Ile Gly Leu Leu Ile Met Gly Phe Leu Val
   545 550 555 560
   Leu Gly Leu Val Pro Phe Leu Met Ile Cys Cys Asp Cys Gly Gly Ala
   565 570 575
   Pro Arg Ser Ala Ala Gly Phe Glu Pro Val Pro Glu Cys Ser Asp Gly
   580 585 590
   Ala Ile His Ser Trp Ala Val Glu Gly Pro Gln Pro Glu Pro Arg Asp
   595 600 605
   Ile Thr Thr Val Ile Pro Gln Ile Pro Pro Asp Asn Ala Asn Ile Ile
   610 615 620
   Glu Cys Ile Asp Asn Ser Gly Val Tyr Thr Asn Glu Tyr Gly Gly Arg
   625 630 635 640
   Glu Met Gln Asp Leu Gly Gly Gly Glu Arg Met Thr Gly Phe Glu Leu
   645 650 655
   Thr Glu Gly Val Lys Thr Ser Gly Met Pro Glu Ile Cys Gln Glu Tyr
   660 665 670
   Ser Gly Thr Leu Arg Arg Asn Ser Met Arg Glu Cys Arg Glu Gly Gly
   675 680 685
   Leu Asn Met Asn Phe Met Glu Ser Tyr Phe Cys Gln Lys Ala Tyr Ala
   690 695 700
   Tyr Ala Asp Glu Asp Glu Gly Arg Pro Ser Asn Asp Cys Leu Leu Ile
   705 710 715 720
   Tyr Asp Ile Glu Gly Val Gly Ser Pro Ala Gly Ser Val Gly Cys Cys
   725 730 735
   Ser Phe Ile Gly Glu Asp Leu Asp Asp Ser Phe Leu Asp Thr Leu Gly
   740 745 750
   Pro Lys Phe Lys Lys Leu Ala Asp Ile Ser Leu Gly Lys Glu Ser Tyr
   755 760 765
   Pro Asp Leu Asp Pro Ser Trp Pro Pro Gln Ser Thr Glu Pro Val Cys
   770 775 780
   Leu Pro Gln Glu Thr Glu Pro Val Val Ser Gly His Pro Pro Ile Ser
   785 790 795 800
   Pro His Phe Gly Thr Thr Thr Val Ile Ser Glu Ser Thr Tyr Pro Ser
   805 810 815
   Gly Pro Gly Val Leu His Pro Lys Pro Ile Leu Asp Pro Leu Gly Tyr
   820 825 830
   Gly Asn Val Thr Val Thr Glu Ser Tyr Thr Thr Ser Asp Thr Leu Lys
   835 840 845
   Pro Ser Val His Val His Asp Asn Arg Pro Ala Ser Asn Val Val Val
   850 855 860
   Thr Glu Arg Val Val Gly Pro Ile Ser Gly Ala Asp Leu His Gly Met
   865 870 875 880
   Leu Glu Met Pro Asp Leu Arg Asp Gly Ser Asn Val Ile Val Thr Glu
   885 890 895
   Arg Val Ile Ala Pro Ser Ser Ser Leu Pro Thr Ser Leu Thr Ile His
   900 905 910
   His Pro Arg Glu Ser Ser Asn Val Val Val Thr Glu Arg Val Ile Gln
   915 920 925
   Pro Thr Ser Gly Met Ile Gly Ser Leu Ser Met His Pro Glu Leu Ala
   930 935 940
   Asn Ala His Asn Val Ile Val Thr Glu Arg Val Val Ser Gly Ala Gly
   945                 950                 955                 960
   Val Thr Gly Ile Ser Gly Thr Thr Gly Ile Ser Gly Gly Ile Gly Ser
                   965                 970                 975
   Ser Gly Leu Val Gly Thr Ser Met Gly Ala Gly Ser Gly Ala Leu Ser
               980                 985                 990
   Gly Ala Gly Ile Ser Gly Gly Gly Ile Gly Leu Ser Ser Leu Gly Gly
           995                 1000                1005
   Thr Ala Ser Ile Gly His Met Arg Ser Ser Ser Asp His His Phe
       1010                1015                1020
   Asn Gln Thr Ile Gly Ser Ala Ser Pro Ser Thr Ala Arg Ser Arg
       1025                1030                1035
   Ile Thr Lys Tyr Ser Thr Val Gln Tyr Ser Lys
       1040                1045
<210> 3
   <211> 8
   <212> PRT
   <213> Homo Sapiens
<400> 3
   Ala Glu Phe His His Ser Ile Met
   1               5
<210> 4
   <211> 12
   <212> PRT
   <213> Homo Sapiens
<400> 4
   Ala Glu Phe His His Ser Ile Met Ser Gln Tyr Lys
   1               5                   10
<210> 5
   <211> 13
   <212> PRT
   <213> Homo Sapiens
<400> 5
   Ala Gly Gln Ile Glu Glu Asn Ser Asn Ala Asn Thr Leu
   1               5                   10
<210> 6
   <211> 14
   <212> PRT
   <213> Homo Sapiens
<400> 6
   Ala Gly Gln Ile Glu Glu Asn Ser Asn Ala Asn Thr Leu Val
   1 5 10
<210> 7
   <211> 16
   <212> PRT
   <213> Homo Sapiens
<400> 7
   Ala Leu Asn Ser Met Gly Gln Asp Leu Glu Arg Pro Leu Glu Leu Arg
   1 5 10 15
<210> 8
   <211> 18
   <212> PRT
   <213> Homo Sapiens
<400> 8
   Ala Ser Ala Ile Ser Val Thr Val Leu Asn Val Ile Glu Gly Pro Val
   1 5 10 15
   Phe Arg
<210> 9
   <211> 22
   <212> PRT
   <213> Homo Sapiens
<400> 9
   Ala Ser Ala Ile Ser Val Thr Val Leu Asn Val Ile Glu Gly Pro Val
   1 5 10 15
   Phe Arg Pro Gly Ser Lys
   20
<210> 10
   <211> 16
   <212> PRT
   <213> Homo Sapiens
<400> 10
   Asp Gly Gly Ala Asp Gly Met Ser Ala Glu Cys Glu Cys Asn Ile Lys
   1 5 10 15
<210> 11
   <211> 17
   <212> PRT
   <213> Homo Sapiens
<400> 11
   Asp Gly Gly Ala Asp Gly Met Ser Ala Glu Cys Glu Cys Asn Ile Lys
   1 5 10 15
   Ile
<210> 12
   <211> 18
   <212> PRT
   <213> Homo Sapiens
<400> 12
   Asp Gly Gly Ala Asp Gly Met Ser Ala Glu Cys Glu Cys Asn Ile Lys
   1 5 10 15
   Ile Leu
<210> 13
   <211> 13
   <212> PRT
   <213> Homo Sapiens
<400> 13
   Asp Ile Asn Asp Asn Pro Pro Val Phe Ser Met Ala Thr
   1 5 10
<210> 14
   <211> 12
   <212> PRT
   <213> Homo Sapiens
<400> 14
   Asp Leu Asp Thr Gly Arg Pro Ser Thr Thr Val Arg
   1 5 10
<210> 15
   <211> 9
   <212> PRT
   <213> Homo Sapiens
<400> 15
   Asp Ser Pro Met Phe Ile Ile Asn Arg
   1 5
<210> 16
   <211> 11
   <212> PRT
   <213> Homo Sapins
<400> 16
   Glu Ile Arg Val Ile Asp Leu Asp Glu Glu Phe
   1 5 10
<210> 17
   <211> 14
   <212> PRT
   <213> Homo Sapiens
<400> 17
   Glu Ile Arg Val Ile Asp Leu Asp Glu Glu Phe Ser Ala Asn
   1 5 10
<210> 18
   <211> 11
   <212> PRT
   <213> Homo Sapiens
<400> 18
   Glu Gln Tyr Gly Gln Tyr Ala Leu Ala Val Arg
   1 5 10
<210> 19
   <211> 9
   <212> PRT
   <213> Homo Sapiens
<400> 19
   Glu Gln Tyr Asn Met Leu Gly Gly Lys
   1 5
<210> 20
   <211> 10
   <212> PRT
   <213> Homo Sapiens
<400> 20
   Glu Ser Ser Asn Val Val Val Thr Glu Arg
   1 5 10
<210> 21
   <211> 11
   <212> PRT
   <213> Homo Sapiens
<400> 21
   Glu Val Thr Pro Phe Phe Ile Ile Tyr Cys Arg
   1 5 10
<210> 22
   <211> 13
   <212> PRT
   <213> Homo Sapiens
<400> 22
   Phe Ile Ser Gly Asn Glu Gly Asn Trp Phe Glu Ile Glu
   1 5 10
<210> 23
   <211> 16
   <212> PRT
   <213> Homo Sapiens
<400> 23
   Phe Leu Val Leu Gly Leu Val Pro Phe Leu Met Ile Cys Cys Asp Cys
   1 5 10 15
<210> 24
   <211> 17
   <212> PRT
   <213> Homo Sapiens
<400> 24
   Phe Leu Val Leu Gly Leu Val Pro Phe Leu Met Ile Cys Cys Asp Cys
   1 5 10 15
   Gly
<210> 25
   <211> 18
   <212> PRT
   <213> Homo Sapiens
<400> 25
   Phe Leu Val Leu Gly Leu Val Pro Phe Leu Met Ile Cys Cys Asp Cys
   1 5 10 15
   Gly Gly
<210> 26
   <211> 17
   <212> PRT
   <213> Homo Sapiens
<400> 26
   Gly Val Gly Ile Asp Gln Pro Pro Tyr Gly Ile Phe Val Ile Asn Gln
   1 5 10 15
   Lys
<210> 27
   <211> 14
   <212> PRT
   <213> Homo Sapiens
<400> 27
   Ile His Ser Asp Cys Ala Ala Asn Gln Gln Val Thr Tyr Arg
   1 5 10
<210> 28
   <211> 16
   <212> PRT
   <213> Homo Sapiens
<400> 28
   Ile Ile Arg Gln Glu Pro Ser Asp Ser Pro Met Phe Ile Ile Asn Arg
   1 5 10 15
<210> 29
   <211> 10
   <212> PRT
   <213> Homo Sapiens
<400> 29
   Ile Leu Asp Val Asn Asp Asn Ile Pro Tyr
   1 5 10
<210> 30
   <211> 10
   <212> PRT
   <213> Homo Sapiens
<400> 30
   Ile Leu Ser Ile Asp Asp Asn Leu Gln Arg
   1 5 10
<210> 31
   <211> 19
   <212> PRT
   <213> Homo Sapiens
<400> 31
   Ile Ser Gly Val Gly Ile Asp Gln Pro Pro Tyr Gly Ile Phe Val Ile
   1 5 10 15
   Asn Gln Lys
<210> 32
   <211> 16
   <212> PRT
   <213> Homo Sapiens
<400> 32
   Lys Ile Ile Arg Gln Glu Pro Ser Asp Ser Pro Met Phe Ile Ile Asn
   1 5 10 15
<210> 33
   <211> 17
   <212> PRT
   <213> Homo Sapiens
<400> 33
   Lys Ile Ile Arg Gln Glu Pro Ser Asp Ser Pro Met Phe Ile Ile Asn
   1 5 10 15
   Arg
<210> 34
   <211> 11
   <212> PRT
   <213> Homo Sapiens
<400> 34
   Lys Leu Lys Ala Ser Ala Ile Ser Val Thr Val
   1 5 10
<210> 35
   <211> 12
   <212> PRT
   <213> Homo Sapiens
<400> 35
   Lys Leu Lys Ala Ser Ala Ile Ser Val Thr Val Leu
   1 5 10
<210> 36
   <211> 8
   <212> PRT
   <213> Homo Sapiens
<400> 36
   Leu Ala Asp Ile Ser Leu Gly Lys
   1 5
<210> 37
   <211> 9
   <212> PRT
   <213> Homo Sapiens
<400> 37
   Leu Asp Arg Glu Gln Tyr Gly Gln Tyr
   1 5
<210> 38
   <211> 11
   <212> PRT
   <213> Homo Sapiens
<400> 38
   Asn Met Leu Gly Gly Lys Tyr Gln Gly Thr Ile
   1 5 10
<210> 39
   <211> 12
   <212> PRT
   <213> Homo Sapiens
<400> 39
   Asn Met Leu Gly Gly Lys Tyr Gln Gly Thr Ile Leu
   1 5 10
<210> 40
   <211> 9
   <212> PRT
   <213> Homo Sapiens
<400> 40
   Asn Asn Phe Leu Asp Arg Glu Gln Tyr
   1 5
<210> 41
   <211> 10
   <212> PRT
   <213> Homo Sapiens
<400> 41
   Asn Asn Phe Leu Asp Arg Glu Gln Tyr Gly
   1 5 10
<210> 42
   <211> 11
   <212> PRT
   <213> Homo Sapiens
<400> 42
   Asn Asn Phe Leu Asp Arg Glu Gln Tyr Gly Gln
   1 5 10
<210> 43
   <211> 12
   <212> PRT
   <213> Homo Sapiens
<400> 43
   Asn Asn Phe Leu Asp Arg Glu Gln Tyr Gly Gln Tyr
   1 5 10
<210> 44
   <211> 8
   <212> PRT
   <213> Homo Sapiens
<400> 44
   Asn Ser Asn Leu Leu Glu Ile Arg
   1 5
<210> 45
   <211> 9
   <212> PRT
   <213> Homo Sapiens
<400> 45
   Asn Val Ile Glu Gly Pro Val Phe Arg
   1 5
<210> 46
   <211> 12
   <212> PRT
   <213> Homo Sapiens
<400> 46
   Asn Val Ile Glu Gly Pro Val Phe Arg Pro Gly Ser
   1 5 10
<210> 47
   <211> 13
   <212> PRT
   <213> Homo Sapiens
<400> 47
   Asn Val Ile Glu Gly Pro Val Phe Arg Pro Gly Ser Lys
   1 5 10
<210> 48
   <211> 14
   <212> PRT
   <213> Homo Sapiens
<400> 48
   Asn Val Ile Glu Gly Pro Val Phe Arg Pro Gly Ser Lys Thr
   1 5 10
<210> 49
   <211> 15
   <212> PRT
   <213> Homo Sapiens
<400> 49
   Asn Val Ile Glu Gly Pro Val Phe Arg Pro Gly Ser Lys Thr Tyr
   1 5 10 15
<210> 50
   <211> 11
   <212> PRT
   <213> Homo Sapiens
<400> 50
   Pro Pro Tyr Gly Ile Phe Val Ile Asn Gln Lys
   1 5 10
<210> 51
   <211> 13
   <212> PRT
   <213> Homo Sapiens
<400> 51
   Gln Glu Pro Ser Asp Ser Pro Met Phe Ile Ile Asn Arg
   1 5 10
<210> 52
   <211> 11
   <212> PRT
   <213> Homo Sapiens
<400> 52
   Arg Ile Ser Gly Val Gly Ile Asp Gln Pro Pro
   1 5 10
<210> 53
   <211> 12
   <212> PRT
   <213> Homo Sapiens
<400> 53
   Arg Ile Ser Gly Val Gly Ile Asp Gln Pro Pro Tyr
   1 5 10
<210> 54
   <211> 13
   <212> PRT
   <213> Homo Sapiens
<400> 54
   Arg Ile Ser Gly Val Gly Ile Asp Gln Pro Pro Tyr Gly
   1 5 10
<210> 55
   <211> 14
   <212> PRT
   <213> Homo Sapiens
<400> 55
   Arg Ile Ser Gly Val Gly Ile Asp Gln Pro Pro Tyr Gly Ile
   1 5 10
<210> 56
   <211> 15
   <212> PRT
   <213> Homo Sapiens
<400> 56
   Arg Ile Ser Gly Val Gly Ile Asp Gln Pro Pro Tyr Gly Ile Phe
   1 5 10 15
<210> 57
   <211> 8
   <212> PRT
   <213> Homo Sapiens
<400> 57
   Ser Ile Asp Asp Asn Leu Gln Arg
   1 5
<210> 58
   <211> 10
   <212> PRT
   <213> Homo Sapiens
<400> 58
   Ser Ile Val Asp Arg Glu Val Thr Pro Phe
   1 5 10
<210> 59
   <211> 12
   <212> PRT
   <213> Homo Sapiens
<400> 59
   Thr Ser Ile Val Asp Arg Glu Val Thr Pro Phe Phe
   1 5 10
<210> 60
   <211> 17
   <212> PRT
   <213> Homo Sapiens
<400> 60
   Thr Ile Glu Ile Gln Glu Asn Thr Leu Asn Ser Asn Leu Leu Glu Ile
   1               5                   10                  15
   Arg
<210> 61
   <211> 10
   <212> PRT
   <213> Homo Sapiens
<400> 61
   Thr Leu Asn Ser Asn Leu Leu Glu Ile Arg
   1               5                   10
<210> 62
   <211> 8
   <212> PRT
   <213> Homo Sapiens
<400> 62
   Thr Met Asn Asn Phe Leu Asp Arg
   1               5
<210> 63
   <211> 13
   <212> PRT
   <213> Homo Sapiens
<400> 63
   Thr Tyr Val Val Thr Gly Asn Met Gly Ser Asn Asp Lys
   1               5                   10
<210> 64
   <211> 12
   <212> PRT
   <213> Homo Sapiens
<400> 64
   Val Ala Thr Asp Leu Asp Thr Gly Arg Pro Ser Thr
   1               5                   10
<210> 65
   <211> 14
   <212> PRT
   <213> Homo Sapiens
<400> 65
   Val Ala Thr Asp Leu Asp Thr Gly Arg Pro Ser Thr Thr Val
   1 5 10
<210> 66
   <211> 15
   <212> PRT
   <213> Homo Sapiens
<400> 66
   Val Ala Thr Asp Leu Asp Thr Gly Arg Pro Ser Thr Thr Val Arg
   1 5 10 15
<210> 67
   <211> 19
   <212> PRT
   <213> Homo Sapiens
<400> 67
   Val Gly Asp Phe Val Ala Thr Asp Leu Asp Thr Gly Arg Pro Ser Thr
   1 5 10 15
   Thr Val Arg
<210> 68
   <211> 11
   <212> PRT
   <213> Homo Sapiens
<400> 68
   Val Ile Asp Leu Asp Glu Glu Phe Ser Ala Asn
   1 5 10
<210> 69
   <211> 29
   <212> PRT
   <213> Homo Sapiens
<400> 69
   Val Ile Gln Pro Thr Ser Gly Met Ile Gly Ser Leu Ser Met His Pro
   1 5 10 15
   Glu Leu Ala Asn Ala His Asn Val Ile Val Thr Glu Arg
   20 25
<210> 70
   <211> 10
   <212> PRT
   <213> Homo Sapiens
<400> 70
   Val Leu Asp Ile Asn Asp Asn Pro Pro Val
   1 5 10
<210> 71
   <211> 13
   <212> PRT
   <213> Homo Sapiens
<400> 71
   Val Leu Asp Ile Asn Asp Asn Pro Pro Val Phe Ser Met
   1 5 10
<210> 72
   <211> 14
   <212> PRT
   <213> Homo Sapiens
<400> 72
   Val Leu Asp Ile Asn Asp Asn Pro Pro Val Phe Ser Met Ala
   1 5 10
<210> 73
   <211> 11
   <212> PRT
   <213> Homo Sapiens
<400> 73
   Val Leu Asn Val Ile Glu Gly Pro Val Phe Arg
   1 5 10
<210> 74
   <211> 10
   <212> PRT
   <213> Homo Sapiens
<400> 74
   Val Met Gly Asn Asn Pro Ala Asp Leu Leu
   1 5 10
<210> 75
   <211> 15
   <212> PRT
   <213> Homo Sapiens
<400> 75
   Val Met Gly Asn Asn Pro Ala Asp Leu Leu Ala Val Asp Ser Arg
   1 5 10 15
<210> 76
   <211> 18
   <212> PRT
   <213> Homo Sapiens
<400> 76
   Val Met Gly Asn Asn Pro Ala Asp Leu Leu Ala Val Asp Ser Arg Thr
   1 5 10 15
   Gly Lys
<210> 77
   <211> 20
   <212> PRT
   <213> Homo Sapiens
<400> 77
   Val Met Gly Asn Asn Pro Ala Asp Leu Leu Ala Val Asp Ser Arg Thr
   1 5 10 15
   Gly Lys Leu Thr
   20
<210> 78
   <211> 20
   <212> PRT
   <213> Homo Sapiens
<400> 78
   Val Val Lys Pro Leu Asp Tyr Glu Ala Met Gln Ser Leu Gln Leu Ser
   1 5 10 15
   Ile Gly Val Arg
   20
<210> 79
   <211> 11
   <212> PRT
   <213> Homo Sapiens
<400> 79
   Val Val Thr Gly Asn Met Gly Ser Asn Asp Lys
   1 5 10
<210> 80
   <211> 15
   <212> PRT
   <213> Homo Sapiens
<400> 80
   Val Val Thr Gly Asn Met Gly Ser Asn Asp Lys Val Gly Asp Phe
   1 5 10 15
<210> 81
   <211> 18
   <212> PRT
   <213> Homo Sapiens
<400> 81
   Val Val Thr Gly Asn Met Gly Ser Asn Asp Lys Val Gly Asp Phe Val
   1 5 10 15
   Ala Thr
<210> 82
   <211> 20
   <212> PRT
   <213> Homo Sapiens
<400> 82
   Val Val Thr Gly Asn Met Gly Ser Asn Asp Lys Val Gly Asp Phe Val
   1 5 10 15
   Ala Thr Asp Leu
   20
<210> 83
   <211> 30
   <212> PRT
   <213> Homo Sapiens
<400> 83
   Val Val Thr Gly Asn Met Gly Ser Asn Asp Lys Val Gly Asp Phe Val
   1 5 10 15
   Ala Thr Asp Leu Asp Thr Gly Arg Pro Ser Thr Thr Val Arg
   20 25 30
<210> 84
   <211> 14
   <212> PRT
   <213> Homo Sapiens
<400> 84
   Tyr Gln Gly Thr Ile Leu Ser Ile Asp Asp Asn Leu Gln Arg
   1 5 10
<210> 85
   <211> 16
   <212> PRT
   <213> Homo Sapiens
<400> 85
   Tyr Val Met Gly Asn Asn Pro Ala Asp Leu Leu Ala Val Asp Ser Arg
   1 5 10 15